# EUROPEAN PATENT APPLICATION

(11) **EP 1 629 783 A1**
(43) Date of publication of application: **01.03.2006**
(21) Application number: 05009383.0
(22) Date of filing: 28.04.2005
(51) Int. Cl.: A61B 17/16

(54) **Self-lubricating surgical instrument**

(30) Priority: 31.08.2004 US 930224
(71) Applicant: Medtronic Inc., Minneapolis, MN 55432-5604 (US)
(72) Inventor: Tidwell, Durell G., Burleson, TY 76028 (US)
(74) Representative: Hughes, Andrea Michelle

(57) **Abstract**

A surgical instrument and a method of lubricating same according to which a vane 32a, b and c is disposed in a chamber defined between a shaft 30 and a lubricant-impregnated housing 20. When air is introduced into the chamber it impinges against the vane and rotates the shaft, and the lubricant weeps onto the inner wall of the housing to lubricate the wall.

## Description

### Field of the Invention

The present invention relates generally to surgical instruments and in particular to surgical instruments for dissecting bone and other tissue.

### Background

Many conventional surgical instruments employ motors to rotate a cutting element for the dissection of bone or other tissue. The motor usually includes a rotary shaft and a dissection tool coupled to the shaft and having a cutting or abrading element that is rotated at relatively high speeds by the motor.

It can be appreciated that these instruments are relatively small yet the motor is required to drive the cutting element at relatively high speeds that generate heat. Thus, the motor must be lubricated, usually from an external source that is connected to the motor via a passage, conduit, or the like, which creates problems in the form of design challenges due to the small size of the instrument.

All patents listed in Table 1 provide background to the present invention.

**Table 1**

| **Patent / Publication No.** | **Patented / Published Date** | **Inventor** |
|---|---|---|
| 4,068,987 | 01/17/1978 | Crooks |
| 4,197,061 | 04/08/1980 | Hill |
| 5,834,870 | 11/10/1998 | Tokushima et al. |
| 6,413,062 | 07/02/2002 | Peters |
| 2002/0151902 A1 | 10/17/2002 | Riedel et al. |
| 2003/0023256 A1 | 01/30/2003 | Estes et al. |
| 2003/0163134 A1 | 08/28/2003 | Riedel et al. |
| 6,626,577 | 09/30/2003 | Homg, et al. |
| 2003/0229351 A1 | 12/1112003 | Tidwell et al. |

### Summary

The present invention eliminates the need for an external lubricant source and any passages, conduits, or the like, for the lubricant by providing a surgical instrument with a motor that is self-lubricating, and provides an instrument and method as defined in the claims.

Various embodiments of the invention discussed below may possess one or more of the above features and advantages, or provide one or more solutions to the above problems existing in the prior art. The specific description is given by way of example only.

### Brief Description of the Drawings

Fig. 1 is an isometric view of a surgical instrument according to an embodiment of the present invention.

Fig. 2 is an enlarged exploded view of the instrument of Fig. 1.

Fig. 3 is an enlarged, partial sectional view of the embodiment of Figs. 1 and 2 shown in an assembled condition.

Fig. 4 is an end view of the components of the instrument of Fig. 4.

### Detailed Description of the Preferred Embodiments

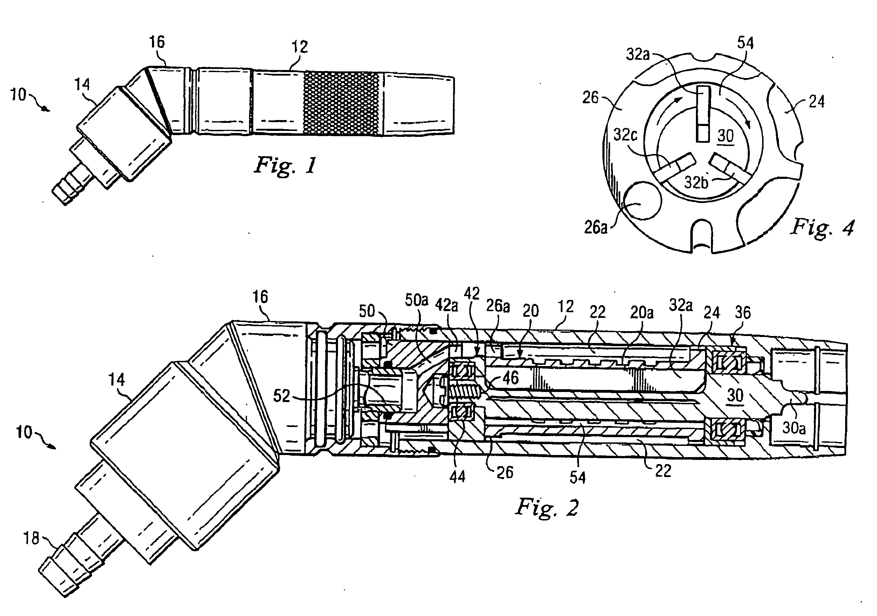

Referring to Figs. 1 and 2 of the drawings, the reference 10 refers, in general, to a surgical instrument according to an embodiment of the invention which includes an outer casing 12 connected to a swivel assembly 14, via a coupler 16. One end of the coupler 16 is in threaded engagement with the rear end of the casing 12 and the other end is connected to one end of the assembly 14 by a conventional swivel connection which will not be disclosed in detail.

An air inlet tube 18 has one end portion projecting from the other end of the assembly 14 for attachment to an air hose (not shown), so that air passes through the assembly 14 and the coupler 16 to the interior of the casing for use in a manner to be described. The front end of the casing 12 is open and is adapted to receive a cutting element (not shown), a portion of which would extend in the casing for connection to the instrument 10 in a manner to be described.

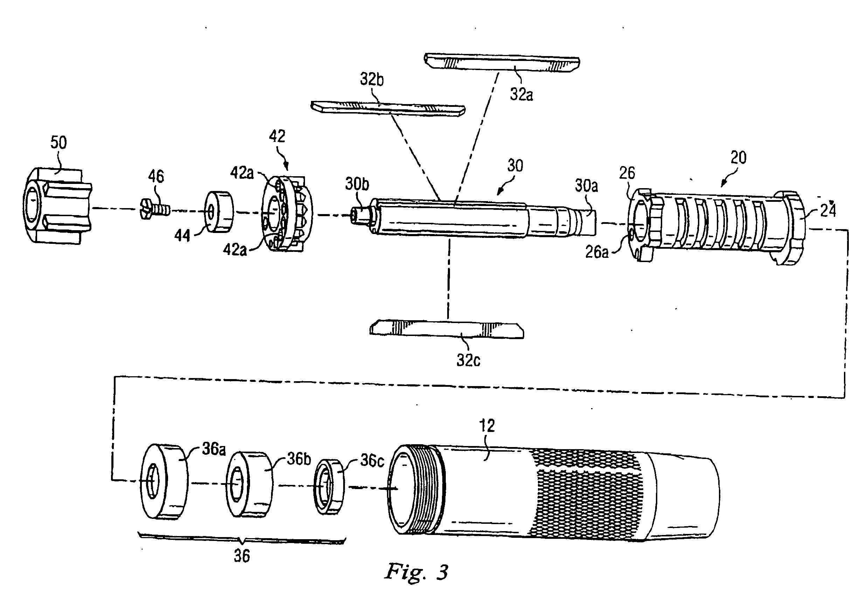

Referring to Figs. 2 and 3, a cylindrical rotor housing 20 is located in the casing 12 with the outer surface of the housing extending in a spaced relation to the inner surface of the casing 12 to define an air chamber 22. Two annular flanges 24 and 26 are formed at the respective ends of the housing 20, and a through opening 26a is formed in the flange 26 for reasons to be described.

The outer diameter of the flanges correspond to the inner diameter of the casing 12 so that the outer surfaces of the flanges engage the inner wall of the casing with minimal clearance to support the rotor housing 20 in the casing. A series of five spaced, parallel arcuate air slots 20a are formed in the housing 20 for permitting the ingress of air into the interior of the housing under conditions to be described.

A shaft 30 is supported in the casing 12 in a manner to be described, and a mounting flange 30a is formed at one end of the shaft 30 that projects from the corresponding end of the housing and is adapted to be engaged by the above-mentioned cutting element (not shown). A reduced-diameter portion 30b is formed at the other end of the shaft 30 and projects out from the other end of the housing 20 for reasons to be described.

Three elongated vanes 32a, 32b and 32c are disposed in three angularly-spaced, longitudinal slots formed in the outer surface of the shaft 30. Portions of the vanes project from the slots and the vanes are adapted for radial movement in the slots under conditions to be described.

A bearing assembly 36 extends in the casing 12 and around the front end portion of the shaft 30. The bearing assembly 36 is conventional and, as such, consists of a housing 36a, a bearing 36b that extends in the housing, and a seal 36c. As shown in Fig. 2, the bearing housing 36a and the bearing 36b are located between the front surface of the flange 24 and a shoulder formed in the interior of the casing 12, and the seal 36c extends in a groove formed in the casing and engages the bearing 36b.

A bearing assembly 40 is also disposed in the casing 12 and extends around the reduced-diameter portion 30b of the shaft 30. The bearing assembly 40 is conventional and, as such, consists of a housing 42 (Fig. 3) and a bearing 44 that extends in the housing. A series of angularly spaced through openings 42a are provided through the housing 42, for reasons to be described. A set screw 46 threadedly engages a threaded opening in the reduced-diameter portion 30b of the shaft 30, with its head engaging the bearing 44 to maintain the assembly 40 in the above position.

The shaft 30 is thus supported for rotation in the casing 12 by the bearing assemblies 36 and 40, with the mounting flange 30a of the shaft 30 located in the interior of the front end portion of the casing 12 so that it can be coupled to a standard cutting tool (not shown) in a conventional manner. Thus, when the shaft 30 is rotated in a manner to be described, it drives the tool.

An annular air distributor 50 is disposed in the casing 12 between the bearing assembly 40 and the rear end of the casing. A tube 52 (Fig.2) extends from the assembly 14 and through the coupler 16 into a central opening in the distributor 50. Thus, air from the assembly 14 (Fig. 1) is passed, via the tube 46, to the distributor 50.

As shown in Fig. 2, an internal air passage 50a is provided in the distributor 50 that connects the air tube 46 to one of the openings 42a of the bearing housing 42. The latter opening is in alignment with the opening 26a in the flange 26 of the housing 20 so that the air passes from the tube 46, through the passage 50a, the openings 42a and 26a, and into the air chamber 22.

As shown in Figs. 2 and 4, the shaft 30 is eccentrically disposed in the housing 20 to define an annular chamber 54 that varies in thickness, or cross section, in an angular direction around the shaft. Thus, as the vanes 32a, 32b, and 32c rotate with the shaft 30 under conditions to be described, the vanes move radially in the above-mentioned slots in the shaft 30 depending on their angular position in the chamber 52.

The rotor housing 20 is manufactured from a lubricant-impregnated material, such as bronze, so that when subjected to relatively high temperatures, the lubricant will "weep" from the material, in a conventional manner. Examples of such a lubricant-impregnated material is a lubricant-impregnated sintered bronze material manufactured and marketed by Anchor Bronze and Metals, Inc. of Cleveland, Ohio, and by Bunting Bearings of Holland, Ohio. Applying this technology to the housing 20, the housing would be manufactured of a bronze, or similar material as specified by these companies and after forming, sintering and sizing, the material is vacuum-impregnated with a lubricant, such as oil, which flows, or weeps, from the material during the operation that will be described.

In operation, a tool is coupled to the mounting flange 30 of the shaft 30 and an air hose is connected to the tube 18 of the assembly 14. The air passes through the later assembly, through the tube 52, the passage 50a, the openings 42a and 26a, and into the air chamber 22. From the chamber 22, the air passes through the slots 20a in the housing 20 and into the chamber 54 where it impinges against the vanes 32a, 32b, and 32c, causing rotation of the shaft 30 to drive the above-mentioned cutting tool. During this action, the vanes 32a, 32b, and 32c are pushed, or forced, radially outwardly against the inner wall of the housing 20 as they rotate with the shaft 30, creating heat and raising the temperature of the wall of the housing 20. This causes the above-mentioned impregnated lubricant to weep from the material forming the housing 20 to the inner wall of the housing, and thus lubricate the interfaces between the housing and the vanes 32a, 32b, and 32c. As a result, adequate lubrication is provided without having to pass lubricant from an external source into the instrument 10.

It is understood that variations may be made in the above without departing from the scope of the invention. For example, the number of vanes and the type of lubricant impregnated into the wall of the housing 20 can be varied. Also, the type of element attached to the rotating shaft and the type of lubricant may be varied. Further, the structure for introducing air into the casing 12 and/or into the housing 20 may be varied. Still further, the shaft 30 can be used to drive any element that may be used in a surgical procedure. Moreover, the specific type of motor used is not limited to a pneumatic motor.

The preceding specific embodiment is illustrative of the practice of the invention. It is to be understood that other expedients known to those skilled in the art or disclosed herein, may be employed without departing from the invention, which is defined by the scope of the appended claims. For example, the present invention is not limited to surgical instruments employing a cutting element, but may find further application in which high speed rotation of a relatively small motor is required.

## Claims

1. A surgical instrument comprising a shaft (30), a housing (20) having a cylindrical wall extending around the shaft in a spaced relation to the shaft for defining a chamber, and at least one vane (32a, 32b, 32c) extending from the shaft so that air introduced into the chamber impinges on the vane and rotates the shaft, the wall being formed by a lubricant-impregnated material that lubricates the wall during the rotation.

2. The instrument of claim 1 wherein the air forces the vane against the inner wall of the housing causing heat, and wherein the lubricant weeps from the housing in response to the heat.

3. The instrument of claim 2 wherein the lubricant lubricates the interface between the wall and the vane.

4. The instrument of any preceding claim wherein the housing is mounted in a casing (12), and wherein the air is introduced into a space defined between the housing and the casing and into the chamber through slots formed in the housing.

5. The instrument of any preceding claim wherein there are three angularly spaced vanes extending between the shaft and the housing.

6. The instrument of any preceding claim wherein a portion of the vane extends in a slot formed in the shaft and the shaft is eccentrically disposed in the housing so that the vane moves radially in the slot during the rotation of the shaft.

7. The instrument of any preceding claim wherein the shaft is adapted to be connected to a tool for performing a surgical procedure.

8. A method of operating and lubricating a surgical instrument comprising impinging air on a vane in a housing to rotate the vane and generate heat, and impregnating a wall of the housing with lubricant that weeps from the wall in response to the heat.

9. The method of claim 8 wherein the air forces the vane against the inner wall of the housing to generate the heat, and wherein the lubricant lubricates the interface between the wall and the vane.

10. The method of claim 8 further comprising mounting a shaft in the housing for rotation with the vane and wherein the step of impinging comprises introducing the air into a chamber defined between the shaft and the housing.

11. The method of claim 10 further comprising mounting the housing in a casing, and wherein the air is introduced into a space defined between the housing and the casing and into the chamber through slots formed in the housing.

12. The method of claim 10 further comprising disposing a portion of the vane in a slot in the shaft and mounting the shaft eccentrically in the housing so that the vane moves radially in the slot during the rotation of the shaft.

13. The method of claim 10 further comprising connecting the shaft to a tool for performing a surgical procedure.

14. A surgical instrument comprising a shaft, a housing having a cylindrical wall extending around the shaft, means for rotating the shaft, and means for responding to heat generated by the rotating shaft and lubricating the housing.

15. The instrument of claim 14 wherein the lubricating means comprises lubricant impregnated in the wall of the housing that weeps on the inner surface of the wall in response to the heat generation.

16. The instrument of claim 14 wherein the housing is spaced from the shaft to define a chamber, and wherein the means for rotating comprises at least one vane extending between the shaft and the housing so that air introduced into the chamber impinges on the vane and rotates the shaft.

17. The instrument of claim 16 wherein the vane extends to the wall and wherein the lubricating means lubricates the interface between the wall and the vane.

18. The instrument of claim 16 wherein the housing is mounted in a casing, and wherein the air is introduced into a space defined between the housing and the casing and into the chamber through slots formed in the housing.

19. The instrument of claim 16 wherein the air forces the vane against the inner wall of the housing causing the heat, and wherein the lubricating means comprises lubricant impregnated in the housing and adapted to weep from the housing in response to the heat to lubricate the interface between the wall and the vane.

20. The instrument of claim 16 wherein the shaft is eccentrically disposed in the housing and wherein a portion of the vane extends in a slot formed in the shaft and moves radially in the slot during the rotation of the shaft.

21. The instrument of claim 14 wherein the shaft is adapted to be connected to a tool for performing a surgical procedure.

22. A surgical instrument comprising a housing having a lubricant-impregnated wall, and a vane adapted for rotation in the housing to generate heat and cause the lubricant to weep on the inner surface of the wall.

23. The instrument of claim 22 wherein the vane extends to the wall and wherein the lubricant lubricates the interface between the wall and the vane.

24. The instrument of claim 22 further comprising a shaft mounted for rotation in the housing and spaced from the housing to define a chamber, and wherein the vane extends from the shaft so that air introduced into the chamber impinges on the vane and rotates the shaft.

25. The instrument of claim 24 wherein the housing is mounted in a casing, and wherein the air is introduced into a space defined between the housing and the casing and into the chamber through slots formed in the housing.

26. The instrument of claim 24 wherein the air forces the vane against the inner wall of the housing to cause the heat.

27. The instrument of claim 24 wherein the shaft is eccentrically disposed in the housing and wherein a portion of the vane is disposed in a slot formed in the shaft and moves radially in the slot during the rotation of the shaft.

28. The instrument of claim 24 wherein the shaft is adapted to be connected to a tool for performing a surgical procedure.

29. A surgical instrument comprising a housing having a lubricant-impregnated wall, and a vane extending to the wall and adapted for rotation in the housing to generate heat and cause the lubricant to weep from the wall and lubricate the interface between the wall and the vane.

30. A method of operating and lubricating a surgical instrument comprising impinging air on a vane in a housing to rotate the vane and push it against the inner wall of the housing to generate heat, and impregnating a wall of the housing with lubricant that weeps from the wall in response to the heat and lubricates the interface between the wall and the vane.

31. A surgical instrument comprising a housing having a lubricant-impregnated wall, a vane adapted for rotation in the housing, means for introducing air into the housing that impinges on the vane to rotate the vane and force it against a wall of the housing to generate heat and cause the lubricant to weep from the wall and lubricate the interface between the wall and the vane, and a shaft mounted for rotation in the housing and connected to the vane so that rotation of the vane rotates the shaft.
